Europäisches Patentamt

European Patent Office

Office européen des brevets

(19)

(11) **EP 1 550 402 A1**

## (12) EUROPEAN PATENT APPLICATION
published in accordance with Art. 158(3) EPC

(43) Date of publication:
06.07.2005 Bulletin 2005/27

(21) Application number: 03754034.1

(22) Date of filing: 08.10.2003

(51) Int Cl.7: **A61B 8/00**

(86) International application number:
**PCT/JP2003/012895**

(87) International publication number:
**WO 2004/032744 (22.04.2004 Gazette 2004/17)**

(84) Designated Contracting States:
**AT BE BG CH CY CZ DE DK EE ES FI FR GB GR HU IE IT LI LU MC NL PT RO SE SI SK TR**

(30) Priority: **08.10.2002 JP 2002294606**

(71) Applicant: **MATSUSHITA ELECTRIC INDUSTRIAL CO., LTD.**
**Kadoma-shi, Osaka 571-8501 (JP)**

(72) Inventor: **FUKUKITA, Hiroshi**
**Setagaya-ku, Tokyo 154-0016 (JP)**

(74) Representative: **Leeming, John Gerard et al**
**J.A. Kemp & Co.,**
**14 South Square,**
**Gray's Inn**
**London WC1R 5JJ (GB)**

### (54) ULTRASONIC DIAGNOSIS DEVICE

(57) An ultrasonic diagnostic apparatus which is capable of generating a pulse sound field with high sensitivity and high resolution, particularly in short distances, is disclosed. In the ultrasonic diagnostic apparatus according to the present invention, a hyperbola operation section 4 derives the distance from each of a plurality of arranged transducer elements 1 to the convergence positions from a hyperbolic function wherein the gradient "a" of an asymptote is $0 < |a| < 1$, with the positions in the horizontal direction of the ultrasonic transducer elements as the variable, and a delay data generation section 3 and a driving circuit 2 generate the driving pulse of each of the plurality of ultrasonic transducer elements delayed in accordance to the distances calculated by the hyperbola operation section.

FIG. 1

EP 1 550 402 A1

**Description**

TECHNICAL FIELD

The present invention relates to an ultrasonic diagnostic apparatus which performs delay control on the ultrasonic beams of a plurality of ultrasonic transducer elements arranged in a horizontal direction to a specimen.

BACKGROUND ART

An ultrasonic diagnostic apparatus of this type is configured to perform delay control on the ultrasonic beams of a plurality of ultrasonic transducer elements arranged in a horizontal direction to a specimen so as to enable all of the ultrasonic beams to be converged on the same focal point. In addition, what is described in the following Patent Reference 1 is known as a conventional ultrasonic diagnostic apparatus. In this conventional ultrasonic diagnostic apparatus, a plurality of ultrasonic transducer elements are classified into a plurality of ultrasonic transducer element groups and configured along the array direction so as to converge the ultrasonic beams on a plurality of different convergence points, the convergence points of the transmitted and received ultrasonic waves are configured to be mutually different according to each of these ultrasonic transducer element groups, and ultrasonic waves having a plurality of convergence points can be received simultaneously.

Patent Reference 1: Japanese Patent Laid-Open Publication No. 55-26976

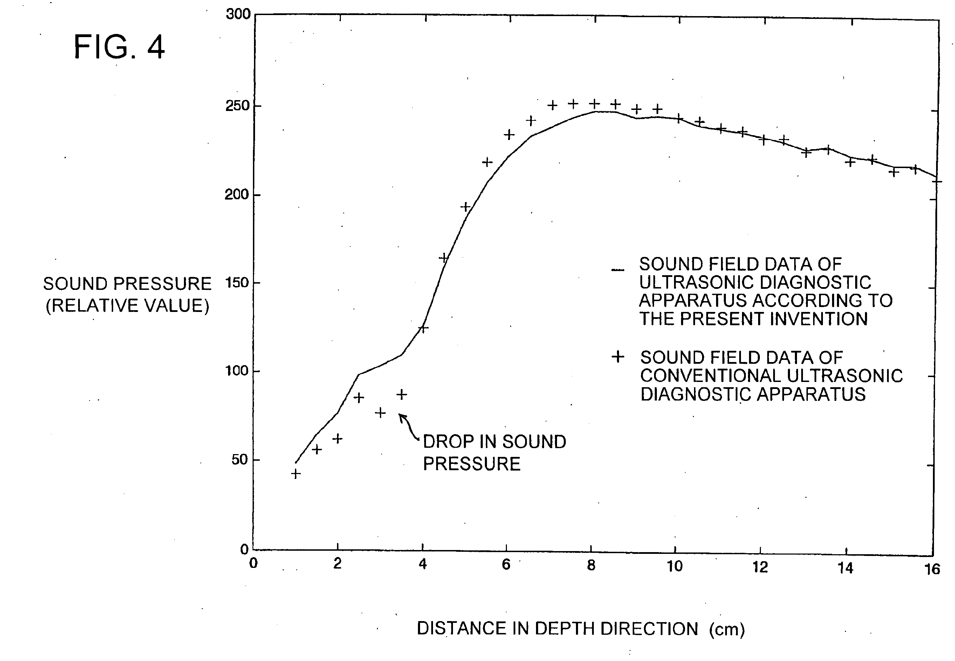

However, in the conventional ultrasonic diagnostic apparatus, there was a problem in that a large number of parameters existed in regards to the classification method of the ultrasonic transducer elements into a plurality of ultrasonic transducer element groups and the designation of the positions of the plurality of convergence points, and the optimization of these parameters was difficult. Here, in Fig. 4, the sound field data of a conventional ultrasonic diagnostic apparatus are plotted and indicated with + mark, and in this example, the sound pressure drops in a short distance of about 2 cm.

DISCLOSURE OF THE INVENTION

The present invention has been made to solve conventional problems and its objective is to provide an ultrasonic diagnostic apparatus wherein the types of parameters for generating delay time are reduced, the sensitivity is high even with few parameters, and the optimization of convergence in short distances, in particular, can be facilitated.

In order to achieve the objectives above, the present invention is configured to comprise, in the ultrasonic diagnostic apparatus which performs delay control on the ultrasonic beams of a plurality of ultrasonic transducer elements linearly arranged in a horizontal direction to a specimen, a means for deriving the distance from each of the afore-mentioned plurality of ultrasonic transducer elements to the afore-mentioned convergence positions through a hyperbolic function wherein the gradient of an asymptote is $0 < |a| < 1$, with the positions in the horizontal direction of the plurality of ultrasonic transducer elements as the variable, and a means for generating the driving pulse of each of the afore-mentioned plurality of ultrasonic transducer elements delayed in accordance with the derived distances.

Through this configuration, the types of parameters for generating delay time can be reduced and the optimization of convergences can be facilitated, even if the convergence positions differ.

In addition, in order to achieve the afore-mentioned objectives, the present invention is configured to comprise, in the ultrasonic diagnostic apparatus which performs delay control on the ultrasonic beams of a plurality of ultrasonic transducer elements arranged on a convex surface in a horizontal direction to a specimen, a means for deriving the distance from each of the afore-mentioned plurality of ultrasonic transducer elements to the afore-mentioned convergence positions from the sum of a hyperbolic function wherein the gradient of an asymptote is $0 < |a| < 1$, with the positions in the horizontal direction of the afore-mentioned plurality of ultrasonic transducer elements as the variable, and the distance from each of the plurality of ultrasonic transducer elements to a reference line to which the ultrasonic transducer element in the center contacts on the afore-mentioned convex surface and a means for generating the driving pulse of each of the plurality of ultrasonic transducer elements delayed in accordance to the derived distances.

Through this configuration, the types of parameters for generating delay time can be reduced and the optimization of convergence can be facilitated, even if the convergence positions by the ultrasonic transducer elements arranged on the convex surface differ.

BRIEF DESCRIPTION OF THE DRAWINGS

Fig. 1 is a block diagram of an ultrasonic diagnostic apparatus in a first embodiment according to the present invention;
Fig. 2 is an explanatory diagram showing the distance to the position of convergence in the ultrasonic diagnostic apparatus in the first embodiment according to the present invention;
Fig. 3 is an explanatory diagram showing the delay data of each transducer element in the ultrasonic diagnostic apparatus in the first embodiment according to the present invention;

Fig. 4 is a graph showing a comparison of the sound field in the ultrasonic diagnostic apparatus in the first embodiment according to the present invention with the sound field data in a conventional ultrasonic diagnostic apparatus;

Fig. 5 is a block diagram of the ultrasonic diagnostic apparatus in a second embodiment according to the present invention; and

Fig. 6 is an explanatory diagram showing the distance from each of a plurality of ultrasonic transducer elements in the ultrasonic diagnostic apparatus in the second embodiment according to the present invention to a reference line to which the ultrasonic transducer element in the center contacts on the convex surface.

BEST MODE FOR CARRYING OUT THE INVENTION

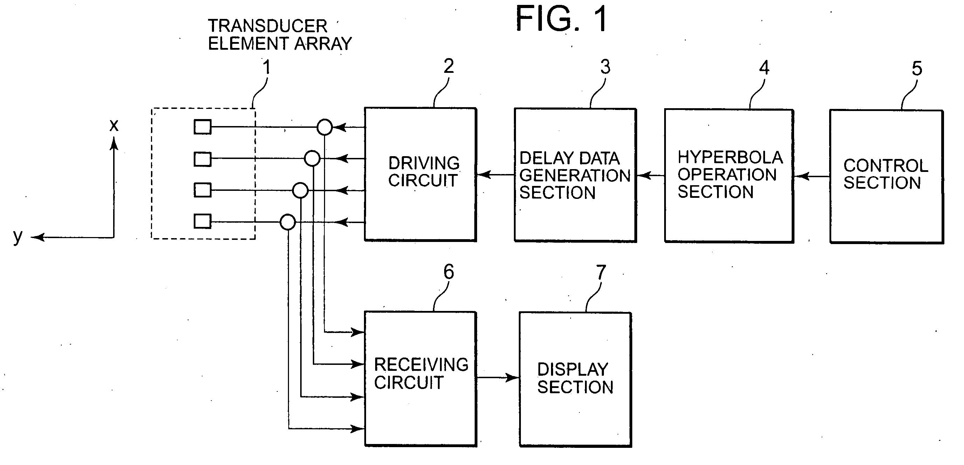

The embodiments according to the present invention will be explained below, with reference to the diagrams. An ultrasonic diagnostic apparatus in a first embodiment according to the present invention is shown in Fig. 1. A transducer element array 1, shown in Fig. 1, is of a linear type wherein a plurality of ultrasonic transducer elements are linearly arranged in a horizontal direction (x direction) to the specimen. A control section 5 provides the gradient "a" of an asymptote in a hyperbola and the curvature "b" in the vicinity of the origin in the hyperbola as the control parameters to a hyperbola operation section 4, and the hyperbola operation section 4 calculates the distance to the convergence positions with each transducer element by the control parameters "a" and "b" and the hyperbola provided by the control section 5. A delay data generation section 3 generates the delay data with each transducer element and provides this data to a driving circuit 2, in accordance to the distance calculated by the hyperbola operation section 4, and the driving circuit 2 drives each transducer element to a timing which is in accordance to the delay data for each transducer element provided by the delay data generation section 3. A receiving circuit 6 performs signal processing on the received signals in the transducer element array 1, and a display section 7 displays the output of the receiving circuit 6.

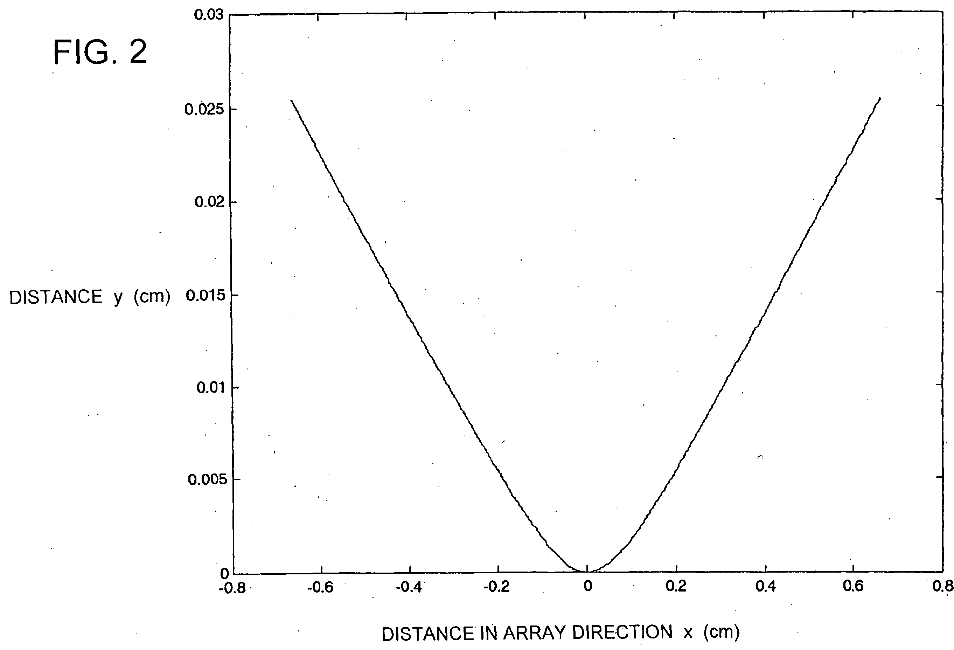

The operations of the ultrasonic diagnostic apparatus, configured as above, are described by using Fig. 1. First, the hyperbola operation section 4 calculates distance y, equivalent to the distance by which an ultrasonic wave such as that shown in Fig. 2 advances, with the following formula (1) based on the gradient "a" of the asymptote in the hyperbola and the curvature "b" in the vicinity of the origin in the hyperbola:

$$(y + b)^2 = (a \, x)^2 + b \qquad (1)$$

The scope of distance x is equivalent to the width of the transmission opening of the transducer element array 1. In the delay data generation section 3, delay data dt (n), corresponding to the n-th transducer element of the transducer element array 1, is calculated as in the following formula (2), with the maximum value of distance y, corresponding to the width of the transmission opening of the transducer element array 1, as y max:

$$dt \, (n) = \{y \, max - y(n)\} \, / \, c \qquad (2)$$

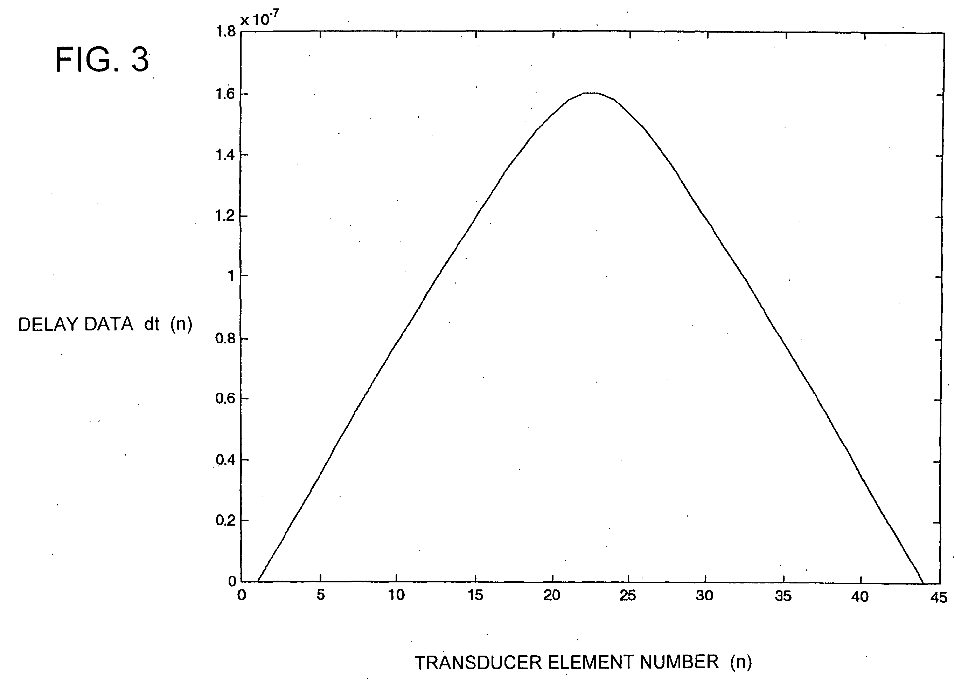

Here, "c" is the sonic speed in the propagation medium. The delay data dt (n), corresponding to the n-th transducer element, is shown in Fig. 3.

The parameters "a" and "b" may be determined independently. However, one parameter can be determined independently, for example, to be 0 < | a | < 1 or to be 0 < b < the distance to the convergent point, first, and the other parameter can be determined next so as to enable the ultrasonic pulse generated by the transducer element located in the center of the transducer element array and the ultrasonic pulses generated by the peripheral transducer elements of the transducer element array to reach the convergent point at the same time, as well.

Fig. 4 is a graph which shows one example of an ultrasonic wave pulse sound field in the depth direction, found through calculation with the delay data dt (n) determined as above, with a solid line and the sound field data of a conventional ultrasonic diagnostic apparatus, plotted with + mark. In this example, distance y is determined to be 8 cm, parameter a = 0.045, and parameter b = 0.005 cm; and there is no drop in sound pressure in short distances in the sound field obtained by the ultrasonic diagnostic apparatus according to the present invention, compared with the sound field obtained by a conventional ultrasonic diagnostic apparatus which has three convergence points. It can be understood through this that, in the ultrasonic diagnostic apparatus according to the present invention, sensitivity is high in short distances and, at the same time, the sound field has a higher horizontal resolution.

The first embodiment according to the present invention shows that, even with few parameters, sensitivity is high in short distances in particular and, at the same time, a sound field with a higher horizontal resolution can be obtained, thereby enabling optimization of the position of convergence, by determining the parameters to be 0 < | a | < 1 and 0 < b< the distance to the convergence point in the formula (1).

< Second Embodiment >

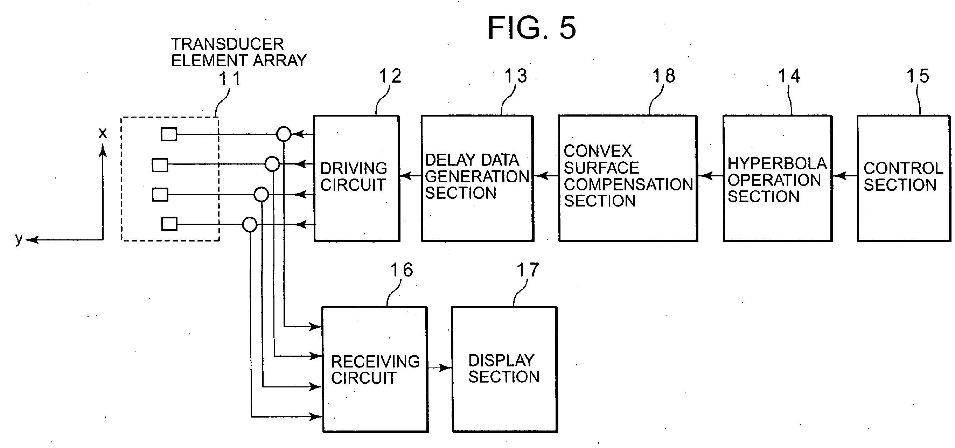

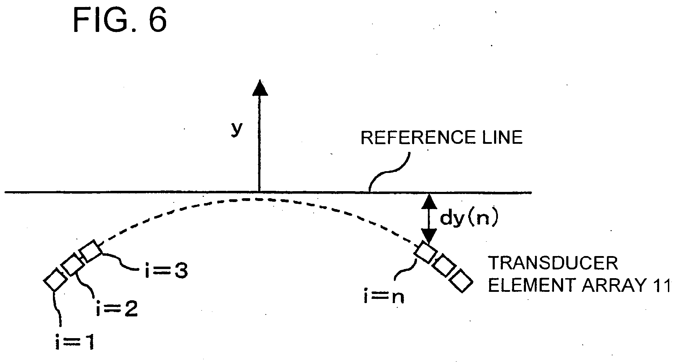

Next, the ultrasonic diagnostic apparatus in a second embodiment according to the present invention is shown in Fig. 5 and Fig. 6. The transducer element array 11 shown in Fig. 5 is a convex type wherein a plurality of transducer elements are arranged on a convex surface in a horizontal direction (x direction) to the specimen, as shown in detail in Fig. 6. A control section 15 provides the gradient "a" of an asymptote in the hyperbola and the curvature "b" in the vicinity of the origin in the hyperbola as the control parameters to a hyperbola operation section 14, and the hyperbola operation section 14 calculates the theoretical distance y, equivalent to the distance to each convergence position of each transducer element based on the control parameters "a" and "b" provided by the control section 15.

As shown in Fig. 6, a convex compensation section 18 provides distance dy (n), from each of the plurality of ultrasonic transducer elements to a reference line to which the ultrasonic transducer element in the center contacts on the convex surface, to a delay data generation section 13. The delay data generation section 13 generates delay data with each transducer element in accordance to the sum of distance y, calculated by the hyperbola operation section 14, and distance dy (n), calculated by the convex surface compensation section 18, and provides this data to a driving circuit 12, and the driving circuit 12 drives each transducer element to a timing which is in accordance to the delay data for each transducer element provided by the delay data generation section 13. A receiving circuit 16 performs signal processing on the received signal of the transducer element array 11 and a display section 17 displays the output of the driving circuit 16.

The operations of the ultrasonic diagnostic apparatus, configured as above, are described by using Fig. 5 and Fig. 6. First, the hyperbola operation section 14 calculates distance y with the formula (1), and in addition, the convex surface compensation section 18 outputs compensated value dy (n) in accordance to each arrangement position of the transducer element array 11. The delay data generation section 13 calculates delay data dt (n), which corresponds to the n-th transducer element, as in the following formula;

$$dt (n)$$

$$= \{ y \, max - y \, (n) - dy \, (n) \} / c \qquad (3)$$

The parameters "a" and "b" may be determined independently. However, one parameter can be determined independently, for example, to be $0 < |a| < 1$ or to be $0 < b <$ the distance to the convergence point, first, and the other parameter may be determined next so as to allow the ultrasonic wave pulse generated by the transducer element in the center of the array and the ultrasonic wave pulses generated by the peripheral transducer elements of the array to reach the convergence point at the same time. As one example, if it is assumed that the distance to the convergent point is 8 cm, parameter a = 0.045, and parameter b = 0.005 cm, as is the case in Fig. 4, there is no drop in sound pressure in short distances in the sound field obtained by the ultrasonic diagnostic apparatus according to the present invention, compared with the sound field obtained by a conventional ultrasonic diagnostic apparatus which has three convergent points. It can be understood through this that, in the ultrasonic diagnostic apparatus according to the present invention, sensitivity is high in short distance and, at the same time, the sound field has a higher horizontal resolution.

The second embodiment according to the present invention such as this shows that, even with few parameters, a sound field with high-sensitivity and, at the same time, high resolution can be obtained, thereby enabling optimization of the convergence position, for the convexly-arranged transducer element array 11, by using the formula (3) and determining the parameters to be $0 < a | < 1$ and $0 < b <$ the distance to the convergence point.

INDUSTRIAL APPLICABILITY

As stated above, according to the present invention, because the distance to a convergence position is derived from a hyperbolic function wherein the gradient "a" of an asymptote is $0 < |a| < 1$, with the positions in the horizontal direction of a plurality of ultrasonic transducer elements as the variable, and the driving pulse of each of the plurality of ultrasonic transducer elements are generated, a sound field which has high sensitivity even in short distances, in particular, and, at the same time, high horizontal resolution can be obtained, thereby enabling optimization of the convergence position, even with few parameters.

**Claims**

1. An ultrasonic diagnostic apparatus for delay-controlling the ultrasonic wave beams of a plurality of ultrasonic transducer elements linearly arranged in a horizontal direction to a specimen, **characterized by**:

   means for deriving the distance from each of said plurality of ultrasonic transducer elements to said convergence positions with from a hyperbolic function wherein the gradient "a" of an asymptote is $0 < |a| < 1$, with the positions in a horizontal direction of said plurality of ultrasonic transducer elements as the variable; and means for generating the driving pulse of each of said plurality of ultrasonic transducer elements delayed in accordance to said derived distances.

2. An ultrasonic diagnostic apparatus for delay-controlling the ultrasonic wave beams of a plurality of ultrasonic transducer elements arranged on a convex surface in a horizontal direction to a specimen, **characterized by**:

means for deriving the distance from each of said plurality of ultrasonic transducer elements to said convergence positions from the sum of a hyperbolic function wherein the gradient "a" of an asymptote is $0 < |a| < 1$, with the positions in a horizontal direction of said plurality of ultrasonic transducer elements as the variable, and the distance from each of said ultrasonic transducer elements and a reference line to which the ultrasonic transducer element in the center contacts on the convex surface; and

means for generating the driving pulse of each of the said plurality of ultrasonic transducer elements delayed in accordance to said derived distances.

# FIG. 1

TRANSDUCER ELEMENT ARRAY

EP 1 550 402 A1

FIG. 2

DISTANCE y (cm)

DISTANCE IN ARRAY DIRECTION x (cm)

FIG. 3

FIG. 4

SOUND PRESSURE (RELATIVE VALUE)

DISTANCE IN DEPTH DIRECTION (cm)

— SOUND FIELD DATA OF ULTRASONIC DIAGNOSTIC APPARATUS ACCORDING TO THE PRESENT INVENTION

+ SOUND FIELD DATA OF CONVENTIONAL ULTRASONIC DIAGNOSTIC APPARATUS

DROP IN SOUND PRESSURE

EP 1 550 402 A1

# FIG. 5

TRANSDUCER
ELEMENT ARRAY
11

x
y

12 DRIVING CIRCUIT

13 DELAY DATA GENERATION SECTION

18 CONVEX SURFACE COMPENSATION SECTION

14 HYPERBOLA OPERATION SECTION

15 CONTROL SECTION

16 RECEIVING CIRCUIT

17 DISPLAY SECTION

EP 1 550 402 A1

## FIG. 6

<table>
<tr><td colspan="2" align="center">**INTERNATIONAL SEARCH REPORT**</td><td>International application No.<br>PCT/JP03/12895</td></tr>
</table>

**A. CLASSIFICATION OF SUBJECT MATTER**
Int.Cl$^7$ A61B8/00

According to International Patent Classification (IPC) or to both national classification and IPC

**B. FIELDS SEARCHED**

Minimum documentation searched (classification system followed by classification symbols)
Int.Cl$^7$ A61B8/00

Documentation searched other than minimum documentation to the extent that such documents are included in the fields searched
| | | | |
|---|---|---|---|
| Jitsuyo Shinan Koho | 1922–1996 | Toroku Jitsuyo Shinan Koho | 1994–2004 |
| Kokai Jitsuyo Shinan Koho | 1971–2004 | Jitsuyo Shinan Toroku Koho | 1996–2004 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 55-26976 A (Tokyo Shibaura Electric Co., Ltd.),<br>26 February, 1980 (26.02.80),<br>Page 3, upper left column, line 5 to page 3, upper right column, line 3; Fig. 1<br>(Family: none) | 1,2 |
| A | JP 59-131338 A (F. Hoffmann-La Roche & Co., AG.),<br>28 July, 1984 (28.07.84),<br>Page 3, lower right column, line 10 to page 4, upper left column, line 19; page 7, upper right column, lines 3 to 20<br>& US 4242912 A & DE 2654280 A1<br>& FR 2445545 A1 | 1,2 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>05 January, 2004 (05.01.04) | Date of mailing of the international search report<br>20 January, 2004 (20.01.04) |
|---|---|
| Name and mailing address of the ISA/<br>Japanese Patent Office | Authorized officer |
| Facsimile No. | Telephone No. |

Form PCT/ISA/210 (second sheet) (July 1998)

**INTERNATIONAL SEARCH REPORT**

International application No.

PCT/JP03/12895

| C (Continuation). DOCUMENTS CONSIDERED TO BE RELEVANT | | |
|---|---|---|
| Category* | Citation of document, with indication, where appropriate, of the relevant passages | Relevant to claim No. |
| A | JP 4-193268 A (Aloka Co., Ltd.), 13 July, 1992 (13.07.92), Page 8, upper right column, line 15 to page 8, lower left column, line 10; Figs. 5, 7 (Family: none) | 1,2 |
| A | JP 10-155793 A (GE Yokogawa Medical Systems, Ltd.), 16 June, 1998 (16.06.98), Column 4, lines 17 to 43; column 6, line 41 to column 7, line 13 (Family: none) | 1,2 |
| A | JP 11-235338 A (GE Yokogawa Medical Systems, Ltd.), 31 August, 1999 (31.08.99), Column 5, lines 38 to 46; Fig. 3 (Family: none) | 1,2 |

Form PCT/ISA/210 (continuation of second sheet) (July 1998)